# EUROPEAN PATENT APPLICATION

(11) **EP 1 769 811 A1**
(43) Date of publication of application: **04.04.2007**
(21) Application number: 05751299.8
(22) Date of filing: 06.06.2005
(51) Int. Cl.: A61M 5/14, A61M 5/142, A61M 5/168

(54) **MEDICINAL LIQUID INJECTION SYSTEM**

(30) Priority: 21.06.2004 JP 2004182475; 11.11.2004 JP 2004327480
(71) Applicant: Nemoto Kyorindo Co., Ltd., Tokyo 113-0033 (JP)
(72) Inventor: NEMOTO, Toru, Nemoto Kyorindo Co., Ltd., Tokyo 1130033 (JP); IKOMA, Tomoyuki, Nemoto Kyorindo Co., Ltd., Tokyo 1130033 (JP); FUKUDA, Takashi, Nemoto Kyorindo Co., Ltd., Tokyo 1130033 (JP)
(74) Representative: Cabinet Plasseraud
(86) International application number: PCT/JP2005/010332
(87) International publication number: WO 2005/123161

(57) **Abstract**

A chemical liquid injection system with which a chemical liquid in a chemical liquid container can be directly injected into a patient without using a n intervening liquid syringe or the like.

A trailing end of chemical liquid injection tube 120 that its leading end is connected to the patient is placed at a lower position within chemical liquid container 200. A leading end of liquid supply tube 110 is placed at a upper position within chemical liquid container 200 and a trailing end of liquid supply tube 110 is connected to liquid container 300. Since liquid pressure-feeding mechanism 131 feeds the liquid in liquid container 300 into chemical liquid container 200 from liquid supply tube 110 with pressure, the chemical liquid is directly injected into the patient from chemical liquid container 200 through chemical liquid tube 120.

## Description

### Technical Field

The present invention relates to a chemical liquid injection system which injects a chemical liquid put in a chemical liquid container into a patient with a chemical liquid injector.

### Background Art

Presently available diagnostic imaging apparatuses for capturing diagnostic images of patients include CT (Computed Tomography) scanners, MRI (Magnetic Resonance Imaging) apparatuses, PET (Positron Emission Tomography) apparatuses, SPECT (Single Photon Emission Computed Tomography) apparatuses, ultrasonic diagnostic apparatuses, angiography apparatuses, MRA (MR angiography) apparatuses and the like.

When the abovementioned diagnostic imaging apparatuses are used, a chemical liquid such as a contrast medium may be injected into a patient. Chemical liquid injectors for automatically performing the injection have been put into practical use. A chemical liquid injector of the type described above includes an injection head on which a liquid syringe is removably mounted.

The liquid syringe comprises a cylinder member formed in a cylindrical shape and a piston member. The cylinder member has a through-hole at its leading end and has an opening at its trailing end. The piston member is slidably inserted into the cylinder member through the opening. Typically, the through-hole at the leading end of the cylinder member is formed as a protruding conduit, and the trailing end of an extension tube is connected to the conduit.

The extension tube is attached integrally to an injection needle at its leading end, and the injection needle is connected to a blood vessel of a patient. When the chemical liquid injector is used, the cylinder member of the liquid syringe filled with a chemical liquid is connected to a patient through the extension tube as described above, and the liquid syringe is mounted on the injection head of the chemical liquid injector.

In a typical chemical liquid injector, the injection head has a concave portion formed in an upper surface in the form fitting the cylinder member of the liquid syringe. The cylinder member is put in the concave portion to hold the liquid syringe. The chemical liquid injector holds the piston member with a piston driving mechanism separately from the cylinder member, and the piston driving mechanism slides the piston member, so that the chemical liquid can be injected into the patient from the liquid syringe.

There are a pre-filled type and a refill type in the liquid syringe. The liquid syringe of the pre-filled type includes a cylinder member filled with a chemical liquid and is wholly sealed by a packing material for shipment. The liquid syringe of the refill type includes a cylinder member which can be filled with a desired chemical liquid by a user. In this case, the liquid syringe of the refill type is connected to a liquid tank of large volume through an extension tube, and the piston member is pulled from the cylinder member of the liquid syringe by a chemical liquid injector or the like to fill the chemical liquid into the liquid syringe from the chemical liquid tank.

When a contrast medium is injected to a patient whose diagnostic image is to be taken by a diagnostic imaging apparatus as described above, the contrast medium is first injected up to a predetermined volume and then physiological saline is injected up to a predetermined volume in many cases. The chemical liquid injector for performing this operation is formed to hold a liquid syringe filled with the contrast medium and a liquid syringe filled with physiological saline in parallel, in which the liquid syringe filled with the contrast medium is first driven and then the liquid syringe filled with the physiological saline is driven. In such a chemical liquid injector, since only the appropriate amount of the contrast medium can be injected into a body part to be imaged, the consumption of the expensive contrast medium can be reduced and the burden on the body of the patient can be alleviated.

In RI (Radio Isotope) tests with the abovementioned PET (Positron Emission Tomography) apparatuses or SPECT (Single Photon Emission Computed Tomography) apparatuses, a chemical liquid with a radioisotope is used as the contrast medium. Since the contrast medium of this type emits harmful radiation, a syringe cover made of tungsten for blocking radiation is put on the liquid syringe to prevent radiation exposure of an operator. Thus, in the chemical liquid injector used in the RI tests, the syringe driving mechanism holds the cylinder member of the liquid syringe covered with the syringe cover.

In the RI tests, since the radioisotope in the contrast medium decays over time, the contrast medium needs to be produced by a dedicated chemical liquid producing apparatus and filled into the liquid syringe immediately before the injection into a patient. In this case, a rubber cap is put on the conduit at the leading end of the liquid syringe, and the sharp injection needle of the chemical liquid injector is inserted into the rubber cap.

Then, the radioisotopic contrast medium is filled into the liquid syringe from the chemical liquid producing apparatus. The operator removes the rubber cap from the liquid syringe to connect the extension tube when the contrast medium is injected into the patient from the liquid syringe. Chemical liquid injectors of the type described above have been invented and applied (see, for example, patent documents 1 and 2 below).
Patent document 1: Japanese Patent Laid-Open No. 2002-11096;
Patent document 2: Japanese Patent Laid-Open No. 2002-102343

### Disclosure of the Invention

### Subject to be solved by the Invention

When the chemical liquid such as the contrast medium is injected into the patient and then the physiological saline is injected as described above, it is necessary to prepare the liquid syringe filled with the chemical liquid and the liquid syringe filled with the physiological saline, and drive these liquid syringes individually. The operation is cumbersome and the structure of the chemical liquid injector is complicated. Malfunctions may occur such as injection of the chemical liquid after injection of the physiological saline.

To inject the liquid in the liquid tank into the patient as described above, the chemical liquid is filled into the liquid syringe of the refill type from the liquid tank and the liquid is injected into the patient from the liquid syringe. The operation is complicated and the chemical liquid is likely to be contaminated during the transfer.

In the abovementioned RI tests, the syringe cover made of tungsten is put on the liquid syringe to prevent radiation exposure of the operator. When the extension tube is connected to the liquid syringe, the operator needs to manually remove the rubber cap from the liquid syringe filled with the radioisotopic contrast medium and connect the extension tube thereto. The operator who connects the extension tube to the liquid syringe is inevitably exposed to radiation.

The present invention has been made in view of the abovementioned problems, and it is an object thereof to provide a chemical liquid injector which can easily realize injection of a chemical liquid such as a contrast medium and then injection of physiological saline into a patient, injection of a chemical liquid in a liquid tank into a patient without using an intervening liquid syringe of the refill type, injection of a liquid without radiation exposure of an operator when a contrast medium with a radioisotope is used, and the like.

### Means to Solve the Subject

A chemical liquid injection system according to a first aspect of the present invention comprises a chemical liquid container and a chemical liquid injector. The chemical liquid injector comprises a chemical liquid injection tube, a liquid supply tube, a liquid container and a liquid pressure-feeding mechanism. The chemical liquid injector contains a chemical liquid. The chemical liquid injector injects the chemical liquid into a patient. The chemical liquid injection tube has a leading end connected to the patient and a trailing end placed at a lower position within the chemical liquid container. The liquid supply tube has a leading end placed at an upper position within the chemical liquid container. The liquid container contains a predetermined liquid and is connected to a trailing end of the liquid supply tube. The liquid pressure-feeding mechanism feeds the liquid in the liquid container to the chemical liquid container from the liquid supply tube with pressure. The liquid is fed to the chemical liquid container with pressure to inject the chemical liquid into the patient from the chemical liquid container through the chemical liquid injection tube.

In a chemical liquid injection system according to a second aspect of the present invention, a liquid supply tube has a leading end placed within a chemical liquid container. Since the chemical liquid container contains a predetermined liquid having a specific gravity smaller than that of the chemical liquid, the liquid is fed to the chemical liquid container with pressure to inject the chemical liquid into the patient from the chemical liquid container through the chemical liquid injection tube.

Various means referred to in the present invention may be arranged to perform their functions, and may comprise dedicated hardware for performing a predetermined function, a chemical liquid injection system whose predetermined function is given by a computer program, a predetermined function performed in a chemical liquid injection system according to a computer program, or a combination thereof. Various components referred to in the present invention do not need to be a separate entity. A plurality of means may be constructed as one member, a certain means may be part of another means, or a certain means may have a portion overlapping a portion of another means.

### Effect of the Invention

In the chemical liquid injection system of the present invention, the liquid pressure-feeding mechanism feeds the liquid in the liquid container from the liquid supply tube to the chemical liquid container with pressure to inject the chemical liquid in the chemical liquid container into the patient from the chemical liquid injection tube. For example, when a contrast medium is used as the chemical liquid and physiological saline is used as the liquid, it is possible to easily and reliably inject the contrast medium and then the physiological saline into the patient, and the chemical liquid can be injected directly into the patient from the chemical liquid container without using an intervening liquid syringe of the refill type or the like. Thus, radiation exposure of the operator can be prevented even when the contrast medium for the RI test is used as the chemical liquid.

### Brief Description of the Drawings

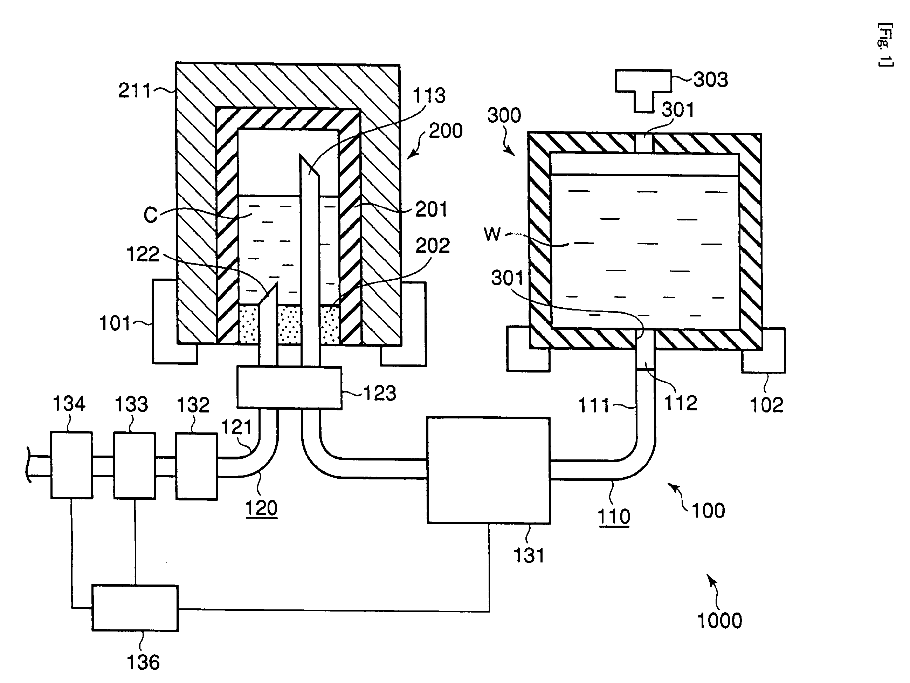

Fig. 1 is a schematic diagram showing a chemical liquid injection system according to an embodiment of the present invention.
Fig. 2 is a schematic diagram showing a chemical liquid injection system according to a first modification.
Fig. 3 is a schematic diagram showing a chemical liquid injection system according to a second modification.
Fig. 4 is a schematic diagram showing a chemical liquid injection system according to a third modification.
Fig. 5 is a schematic diagram showing a chemical liquid injection system according to a fourth modification.
Fig. 6 is a schematic diagram showing a chemical liquid injection system according to a fifth modification.
Fig. 7 is a schematic perspective view showing a modification of a needle member.
Fig. 8 is a schematic diagram showing a chemical liquid injection system according to a sixth modification.
Fig. 9 is a schematic perspective view showing a modification of a needle member.

### Description of Reference Numerals

- 100, 400, 500, 600, 700, 800, 900: CHEMICAL LIQUID INJECTOR
- 101: CHEMICAL LIQUID HOLDING MECHANISM
- 110, 510: LIQUID SUPPLY TUBE
- 113: LONG NEEDLE MEMBER
- 120: CHEMICAL LIQUID INJECTION TUBE
- 122: SHORT NEEDLE MEMBER
- 131: LIQUID PRESSUER-FEEDING MECHANISM
- 200: CHEMICAL LIQUID CONTAINER
- 201: CONTAINER BODY
- 202: ELASTIC MEMBER
- 300: LIQUID CONTAINER
- 410: SHIELD COVER
- 420: EXHAUSTION DETECTION SENSOR
- 430, 513, 514: ONE-WAY VALVE
- 520: LIQUID SYRINGE serving as liquid pressure-feeding mechanism
- 601: CHANGE DETECTION SENSOR
- 1000: CHEMICAL LIQUID INJECTION SYSTEM
- C: CONTRAST MEDIUM serving as chemical liquid
- W: PHYSIOLOGICAL SALINE serving as liquid

### Best Mode for Carrying the Invention

### [Configuration of Embodiment]

An embodiment of the present invention will hereinafter be described with reference to drawings. Chemical liquid injection system 1000 of the present invention comprises chemical liquid injector 100 and replaceable chemical liquid container 200 which contains contrast medium C as a chemical liquid for PET.

More specifically, chemical liquid container 200 comprises cylindrical glass container body 201 having an opening formed at one end. The opening of container body 201 is sealed by elastic member 202 made of silicone rubber or the like. Since contrast medium C for PET is a radioactive material, container cover 211 made of tungsten for blocking the radiation is put outside container body 201.

Chemical liquid injector 100 has liquid container 300 which is replaceably provided for chemical liquid injector 100 separately from chemical liquid container 200. Liquid container 300 contains physiological saline W which is a liquid. Liquid container 300 comprises cylindrical glass container body 302 having opening hole 301 with a small diameter formed at the center of each end. Lid 303 made of silicone rubber or the like is removably put on opening hole 301.

Contrast medium C is a liquid having a specific gravity significantly larger than that of physiological saline W. Chemical liquid container 200 contains contrast medium C of the volume which is to be injected into a patient, and liquid container 300 contains physiological saline W of the volume which is added the volume to be injected into the patient to equal to or more than the same amount as contrast medium C.

Chemical liquid injector 100 of the embodiment has chemical liquid holding mechanism 101 and liquid holding mechanism 102. Chemical liquid holding mechanism 101 holds chemical liquid container 200 such that elastic member 202 is located at the bottom, while liquid holding mechanism 102 holds liquid container 300 such that one of opening holes 301 at both ends is located at the bottom.

Chemical liquid injector 100 of the embodiment has liquid supply tube 110 and chemical liquid injection tube 120 which have tube bodies 111 and 121 made of flexible silicone rubber or the like, respectively, as main portions. Liquid supply tube 110 connects liquid container 300 to chemical liquid container 200, while chemical liquid injection tube 120 connects chemical liquid container 200 to a blood vessel of a patient (not shown).

More specifically, liquid supply tube 110 is integrally attached to hard connector 112 at the trailing end of tube body 111 and removably fitted into opening hole 301 at the bottom of liquid container 300. Liquid supply tube 110 is integrally attached to long, sharp tubular needle member 113 made of metal at the leading end of tube body 111. Long needle member 113 is stuck through elastic member 202 of chemical liquid container 200.

Chemical liquid injection tube 120 is integrally attached to short, sharp tubular needle member 122 made of metal at the trailing end of tube body 121. Short needle member 122 is stuck through elastic member 202 of chemical liquid container 200. The leading end of tube body 121 is integrally attached to a catheter or the like (not shown), and inserted into a blood vessel of a patient for connection.

Since the trailing ends of long needle member 113 and short needle member 122 are held together by holder member 123, long needle member 113 and short needle member 122 are similarly stuck through elastic member 202 of chemical liquid container 200. Thus, when long needle member 113 and short needle member 122 are stuck from below through elastic member 202 of held chemical liquid container 200, the tip of long needle member 113 is located at an upper position within chemical liquid container 200, but the tip of short needle member 122 is located at a lower position within chemical liquid container 200.

The middle portion of liquid supply tube 110 passes through liquid pressure-feeding mechanism 131 which feeds physiological saline W in liquid container 300 into chemical liquid container 200 from liquid supply tube 110 with pressure. Liquid pressure-feeding mechanism 131 has a peristaltic finger mechanism which moves peristaltically, a roller pump mechanism which rotates a plurality of roller members (not shown) or the like, sequentially presses liquid supply tube 110 from the outside to feed physiological saline W with pressure without contact.

Chemical liquid injection tube 120 is connected at the portion near the trailing end thereof to bubble removal mechanism 132, bubble detection sensor 133, and injection block mechanism 134 in order. Bubble detection sensor 133 and injection block mechanism 134 are connected to injection control unit 136 serving as an injection control means together with liquid pressure-feeding mechanism 131.

Bubble removal mechanism 132 comprises a one-way valve or the like and removes any bubble from contrast medium C flowing in chemical liquid injection tube 120. Bubble detection sensor 133 comprises a light-emitting element, a light-receiving element, a processing circuit and the like, and detects any bubble mixed in contrast medium C flowing in chemical liquid injection tube 120.

Injection block mechanism 134 comprises a shutter mechanism which can open and close, and presses chemical liquid injection tube 120 from the outside to block the flow of contrast medium C. Injection control unit 136 comprises a microcomputer or the like on which an appropriate computer program is installed. When any bubble is detected by bubble detection sensor 133, injection control unit 136 forcedly stops liquid pressure-feeding mechanism 131 and causes injection block mechanism 134 to shut chemical liquid injection tube 120.

### [Operation of the Embodiment]

In the structure as described above, when chemical liquid injection system 1000 of the embodiment is used to inject contrast medium C into a patient, chemical liquid container 200 which contains contrast medium C is first prepared. As described above, since contrast medium C for PET is a radioactive material, container body 201 made of glass is placed within container cover 211 made of tungsten.

In currently typical chemical liquid container 200, an open/close cover (not shown) made of tungsten is removably put on the opening of container cover 211. When the open/close cover is removed, elastic member 202 of container body 201 is exposed. In this state, long/short needle members 113 and 122 of liquid supply/chemical liquid injection tubes 110 and 120 of chemical liquid injector 100 are stuck through elastic member 202 of chemical liquid container 200, and then chemical liquid container 200 is held by liquid holding mechanism 101. Then, the tip of long needle member 113 is located at an upper position within chemical liquid container 200, and the tip of short needle member 122 is located at a lower position within chemical liquid container 200.

Next, liquid container 300 which contains physiological saline W and has lids 303 put on opening holes 301 at both ends is prepared. With one of the ends located at the top, lid 303 at the top is removed to expose opening hole 301. In this state, the trailing end of liquid supply tube 110 is connected to opening hole 301 at the top of liquid container 300, and then liquid container 300 is held by liquid holding mechanism 102 with that opening hole 301 located at the bottom. Lid 303 is removed from opening hole 301 located at the top of liquid container 300. Then, physiological saline W in liquid container 300 can be freely supplied to liquid supply tube 110.

In the abovementioned state, for example, when "perform preparatory operation" is input to injection control unit 136, injection control unit 136 opens injection block mechanism 134 and drives liquid pressure-feeding mechanism 131 up to a predetermined time. Then, liquid pressure-feeding mechanism 131 supplies a predetermined volume of physiological saline W from liquid container 300 to chemical liquid container 200 through liquid supply tube 110, so that a predetermined volume of contrast medium C is injected and filled into chemical liquid injection tube 120 from sealed chemical liquid container 200.

When liquid supply/chemical liquid injection tubes 110 and 120 are filled with physiological saline W and contrast medium C, respectively, and air is removed therefrom in this manner, the leading end of chemical liquid injection tube 120 is connected to a blood vessel of a patient. In this state, for example when "perform injection operation" is input to injection control unit 136, injection control unit 136 drives liquid pressure-feeding mechanism 131 up to a predetermined time.

Then, liquid pressure-feeding mechanism 131 supplies a predetermined volume of physiological saline W from liquid container 300 to chemical liquid container 200 through liquid supply tube 110, and contrast medium C is injected into the patient from chemical liquid container 200 through chemical liquid injection tube 120. Since physiological saline W has a specific gravity smaller than that of contrast medium C, contrast medium C is located in a lower portion and physiological saline W is located in an upper portion within chemical liquid container 200.

In chemical liquid injector 100 of the embodiment, since liquid pressure-feeding mechanism 131 is driven up to a predetermined time even after all of contrast medium C is injected to the patient from chemical liquid container 200, physiological saline W fed into chemical liquid container 200 from liquid container 300 with pressure is injected into the patient.

Since contrast medium C is pushed by physiological saline W, contrast medium C reaches the body part of the patient to be imaged by a PET apparatus (not shown) serving as a diagnostic imaging apparatus. Contrast medium C reaching the body part to be imaged is more absorbed by so-called "cancer cells" than by "normal cells," and the absorption of contrast medium C is imaged by the PET apparatus serving as the diagnostic imaging apparatus.

When contrast medium C or physiological saline W is injected into the patient through chemical liquid injection tube 120, any bubble mixed therein is removed by bubble removal mechanism 132. If a bubble is not removed, the bubble is detected by bubble detection sensor 133. In response thereto, injection control unit 136 forcedly stops liquid pressure-feeding mechanism 131 and causes injection block mechanism 134 to shut chemical liquid injection tube 120.

### [Effect of the Embodiment]

In chemical liquid injection system 1000 of the embodiment, physiological saline W in liquid container 300 is fed into chemical liquid container 200 through liquid supply tube 110 with pressure by liquid pressure-feeding mechanism 131, so that contrast medium C in chemical liquid container 200 is injected to the patient from chemical liquid injection tube 120. Thus, contrast medium C can be directly injected into the patient from chemical liquid container 200 without using an intervening liquid syringe of the refill type or the like.

Thus, the operator does not need to fill contrast medium C from chemical liquid container 200 to a liquid syringe or manipulate the liquid syringe. When radioactive contrast medium C is used, radiation exposure of the operator can be avoided favorably. Contamination of contrast medium C can be simply and reliably prevented.

In particular, since the medium for pushing contrast medium C is physiological saline W, contrast medium C can be pushed by that medium which causes no problem when it is injected into the patient. Contrast medium C has a significantly larger specific gravity than that of physiological saline W, and the leading end of liquid supply tube 110 is located at the upper position within chemical liquid container 200 and the trailing end of chemical liquid injection tube 110 is located at the lower position within chemical liquid container 200, so that it is easy to inject only contrast medium C into the patient first and physiological saline W afterward.

In chemical liquid injection system 1000 of the embodiment, the opening of chemical liquid container 200 is sealed by elastic member 202, and long/short needle members 113 and 122 of liquid supply/chemical liquid injection tubes 110 and 120 are stuck through elastic member 202. As a result, liquid supply/chemical liquid injection tubes 110 and 120 can be easily connected to chemical liquid container 200 to favorably prevent radiation exposure of the operator.

Since long/short needle members 113 and 122 of liquid supply/chemical liquid injection tubes 110 and 120 are held together by holder member 123, liquid supply/chemical liquid injection tubes 110 and 120 can be connected easily to chemical liquid container 200, and the tips of long/short needle members 113 and 122 can be readily and reliably placed at appropriate positions within chemical liquid container 200.

When contrast medium C or physiological saline W is injected into the patient through chemical liquid injection tube 120, a bubble mixed therein is removed by bubble removal mechanism 132 to prevent a bubble from being injected into the patient. Any bubble which is not removed by bubble removal mechanism 132 is then detected by bubble detection sensor 133. In response to the detection, injection control unit 136 forcedly stops liquid pressure-feeding mechanism 131, and causes injection block mechanism 134 to shut chemical liquid injection tube 120, so that injection of a bubble into the patient can be eliminated reliably.

### [Modifications of the Embodiment]

The present invention is not in any way limited to the abovementioned embodiment, but various changes and modifications may be made therein without departing from the scope of the invention. For example, in the above embodiment, to inject contrast medium C and physiological saline W favorably with no mixing, short needle member 122 is put on the trailing end of chemical liquid injection tube 120 and long needle member 113 is put on the leading end of liquid supply tube 110 to locate the tailing end of chemical liquid injection tube 120 at the lower position within chemical liquid container 200 and locate the leading end of liquid supply tube 110 at the upper position within chemical liquid container 200.

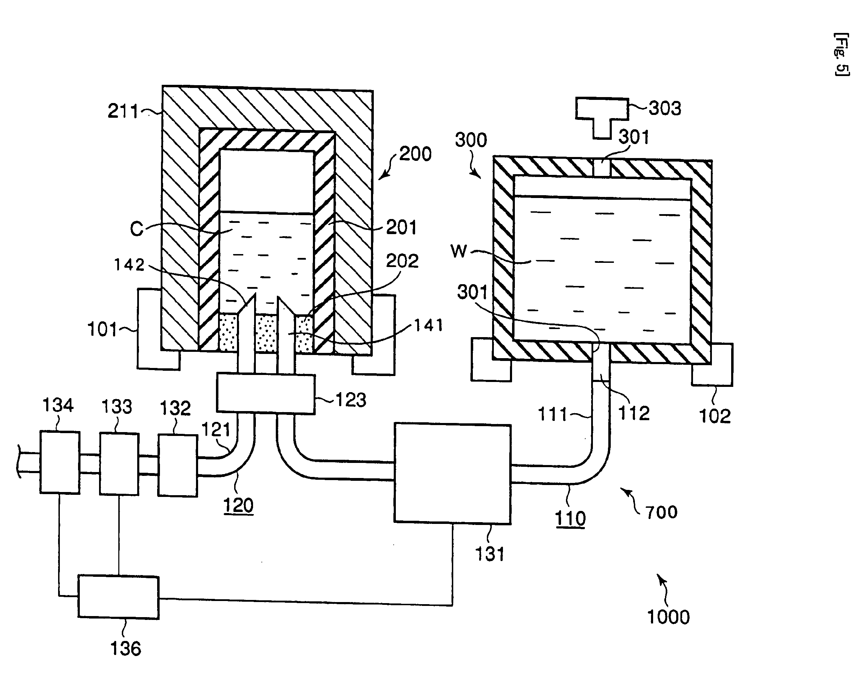

Alternatively, as chemical liquid injector 700 illustrated in Fig. 5, needle-shaped members 141 and 142 of the same length may be put on the leading end of liquid supply tube 110 and the trailing end of chemical liquid injection tube 120 such that both of the leading end of liquid supply tube 110 and the trailing end of chemical liquid injection tube 120 are located at lower positions within chemical liquid container 200.

In this case, the liquid is more likely to mix in the chemical liquid injected into the patient from the beginning. However, if the chemical liquid has a sufficiently larger specific gravity than that of the liquid, or if needle-shaped members 141 and 142 are significantly spaced from each other in the horizontal direction, it is possible to inject the chemical liquid first and the liquid afterward into the patient.

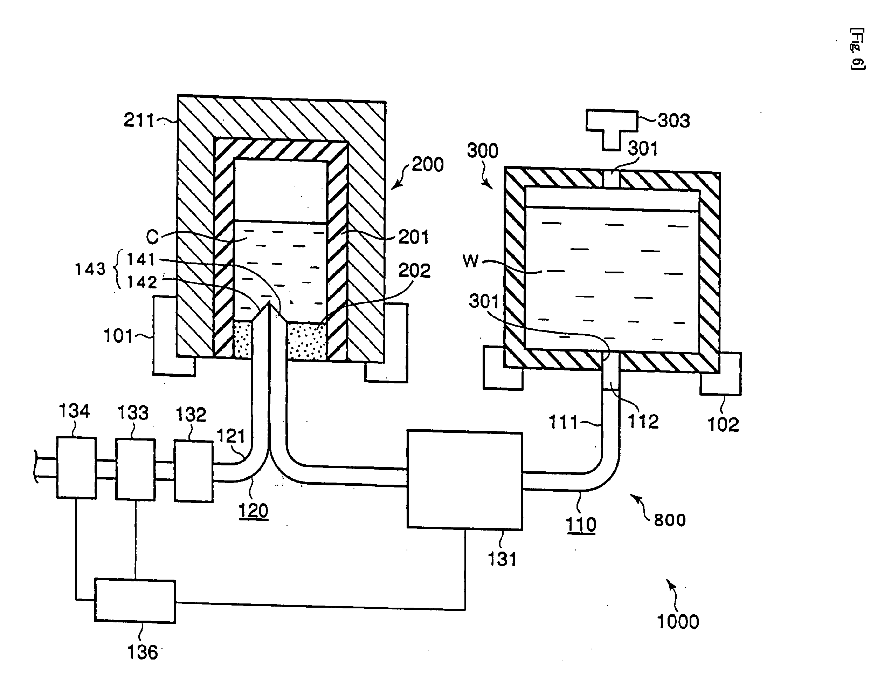

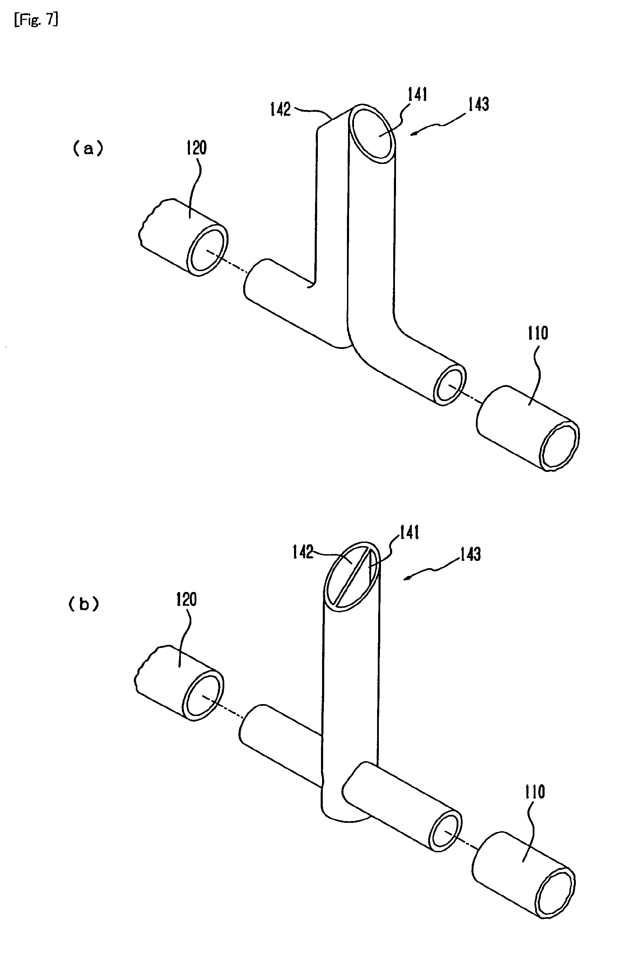

In addition, as shown in chemical liquid injector 800 illustrated in Fig. 6, needle-shaped members 141 and 142 of the same length of liquid supply tube 110 and chemical liquid injection tube 120 may be formed into one needle-shaped member 143. In this case, it is possible to bond two cylindrical needle-shaped members 141 and 142 in parallel as shown in Fig. 7(a) or to form two hollows serving as needle-shaped members 141 and 142 within one cylindrical needle-shaped member 143 in parallel as shown in Fig. 7(b).

In needle-shaped member 143 as described above, holder member 123 for holding two needle-shaped members 141 and 142 together can be omitted and only one needle-shaped member 143 may be stuck through elastic member 202 of chemical liquid container 200, thereby improving the workability.

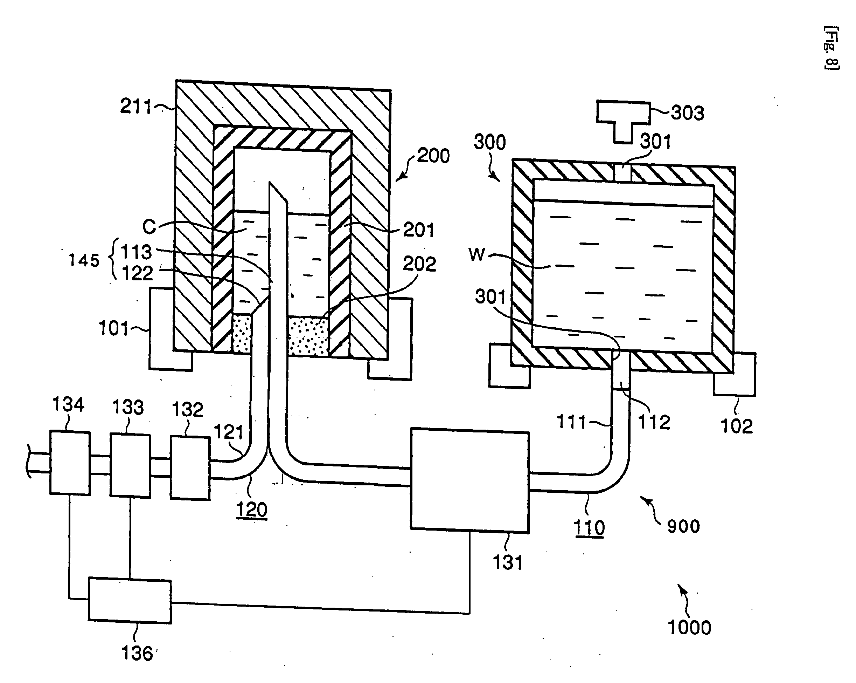

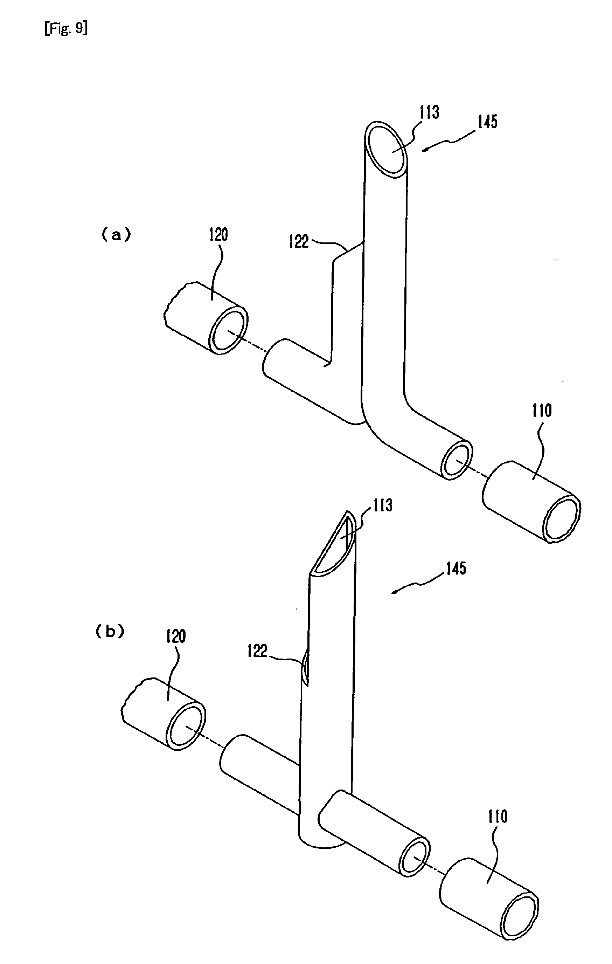

It goes without saying that, as in chemical liquid injector 900 illustrated in Fig. 8, short needle member 122 and long needle member 113 can be formed as one needle-shaped member 145. In this case, as shown in Fig. 9, it is possible to bond cylindrical short needle member 122 and long needle member 113 in parallel or to form two hollows serving as short needle member 122 and long needle member 113 in parallel within one cylindrical needle-shaped member 145.

In the above embodiment, the chemical liquid is realized by the radioactive contrast medium C for PET, but chemical liquid injection system 1000 can be used to inject various types of chemicals. Although container cover 211 of chemical liquid container 200 is made of tungsten in the above embodiment, it may be made of lead.

In the above embodiment, to locate the trailing end of chemical liquid injection tube 120 at the lower position within chemical liquid container 200 and locate the leading end of liquid supply tube 110 at the upper position within chemical liquid container 200, short needle member 122 of chemical liquid injection tube 120 and long needle member 113 of liquid supply tube 110 are stuck through elastic member 202 of chemical liquid container 200, and chemical liquid container 200 is held with elastic member 202 located at the bottom.

Alternatively, it is possible that the long needle member is formed at the trailing end of the chemical liquid injection tube and the long needle member is formed at the leading end of the liquid supply tube (not shown), chemical liquid container 200 is held with elastic member 202 located at the top, and the long needle member of the chemical liquid injection tube and the long needle member of the liquid supply tube are inserted into elastic member 202.

In the above embodiment, to easily connect chemical liquid injection tube 120 and liquid supply tube 110 to existing chemical liquid container 200 for PET, short needle member 122 of chemical liquid injection tube 120 and long needle member 113 of liquid supply tube 110 are stuck through elastic member 202 of chemical liquid container 200. Alternatively, it is possible that short needle member 122 of chemical liquid injection tube 120 and long needle member 113 of liquid supply tube 110 are previously connected to a dedicated elastic member (not shown) and the elastic member is replaced with elastic member 202 of chemical liquid container 200.

More specifically, the opening of the chemical liquid container is sealed to open and close by a sealing member, a short tubular member is formed integrally at the trailing end of the chemical liquid injection tube, and a long tubular member is formed at the leading end of the liquid supply tube. A tube holding member which removably seals the opening of the chemical liquid container is formed. The short tubular member and the long tubular member are formed integrally with the tube holding member (not shown).

In this case, when the sealing member of the chemical liquid container is opened and the tube holding member is attached, and the chemical liquid container is held with the opening located at the bottom. Then, contrast medium C can be injected into the patient if physiological saline W is fed with pressure. In this structure, since the sealing member of the chemical liquid injector is opened and the tube holding member is attached, the operation is more complicated than the insertion of short/long needle members 122 and 113, but it is possible to improve the hermeticity of the chemical liquid container on which pressure is applied.

It goes without saying that, when the abovementioned chemical liquid container is held with the opening located at the top, it is preferable to form the long tubular member integrally at the trailing end of the chemical liquid injection tube and form the short tubular member at the leading end of the liquid supply tube (not shown).

In the above embodiment, liquid supply/chemical liquid injection tubes 110 and 120 are connected to liquid/chemical liquid containers 300 and 200 and then liquid supply/chemical liquid injection tubes 110 and 120 are filled with physiological saline W and contrast medium C to remove air. Alternatively, liquid supply/chemical liquid injection tubes 110 and 120 may be filled with physiological saline W or the like before the connection to liquid/chemical liquid containers 300 and 200.

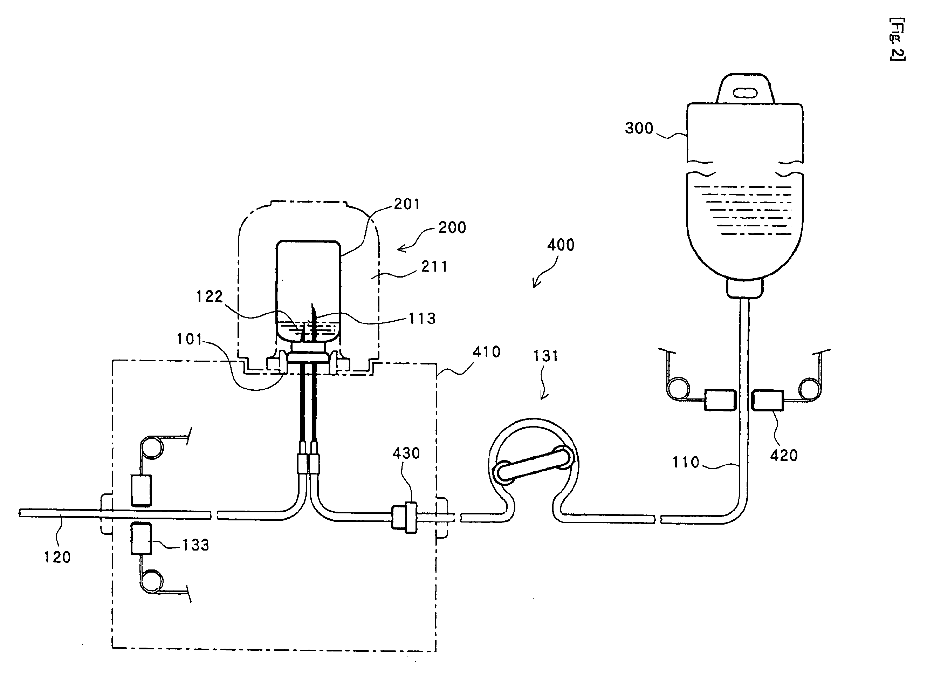

In the above embodiment, only container body 201 of chemical liquid container 200 is shielded by container cover 211. Alternatively, as in chemical liquid injector 400 illustrated in Fig. 2, shield cover 410 for blocking radiation may be formed integrally with chemical liquid holding mechanism 101, and shield cover 410 may cover part of chemical liquid injection tube 120 connected to the trailing end.

In this case, when chemical liquid container 200 to which chemical liquid injection tube 120 is connected is held by chemical liquid holding mechanism 101, only the leading end of chemical liquid injection tube 120 necessary for the connection to the patient is exposed from shield cover 401, so that most of radiation from contrast medium C can be blocked by shield cover 410.

In the above embodiment, when bubble detection sensor 133 detects any bubble mixed in contrast medium C, liquid pressure-feeding mechanism 131 is forcedly stopped, and injection block mechanism 134 shuts chemical liquid injection tube 120. Alternatively, as in chemical liquid injector 400 illustrated in Fig. 2, it is possible to mount exhaustion detection sensor 420 for detecting the exhaustion of physiological saline W on liquid supply tube 110 and forcedly stop liquid pressure-feeding mechanism 131 when exhaustion detection sensor 420 detects the exhaustion, or to shut liquid supply tube 110 by a dedicated supply block mechanism (not shown).

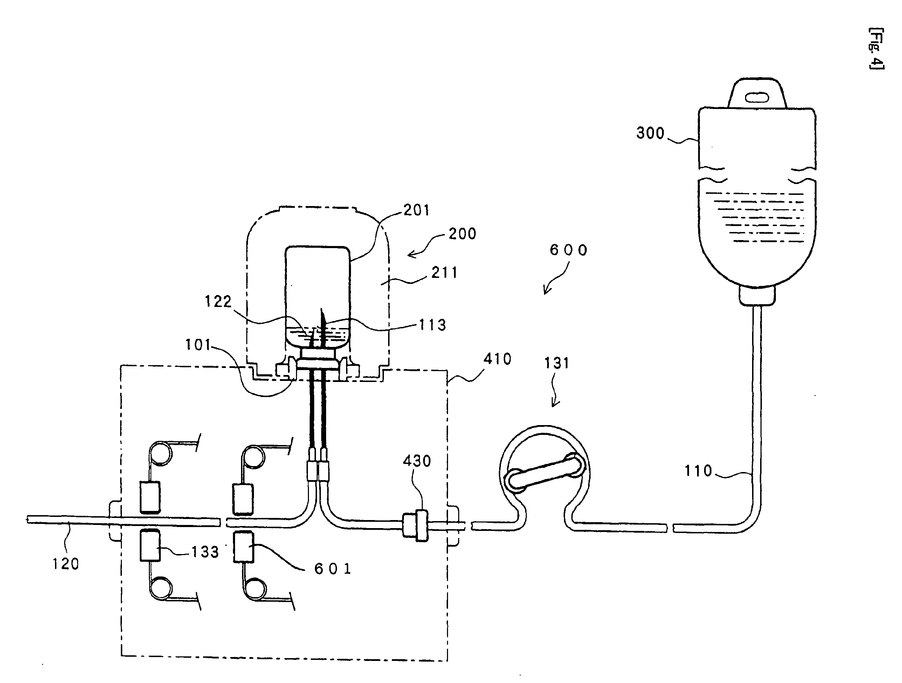

Alternatively, as in chemical liquid injector 600 illustrated in Fig. 4, it is possible to mount change detection sensor 601 on chemical liquid injection tube 120 for detecting the change of radioactive contrast medium C to physiological saline W based on the presence or absence of radiation, and to forcedly stop liquid pressure-feeding mechanism 131 when the change is detected in chemical liquid injection tube 120.

In chemical liquid injector 100 of the embodiment, since contrast medium C in chemical liquid container 200 is pushed by physiological saline W and injected into the patient, it is difficult to appropriately check whether or not all of contrast medium C in chemical liquid container 200 is injected into the patient. However, when change detection sensor 601 detects change of contrast medium C flowing in chemical liquid injection tube 120 to physiological saline W as described above, it is possible to easily and reliably check whether or not all of contrast medium C in chemical liquid container 200 is injected into the patient, and liquid pressure-feeding mechanism 131 can be stopped in desired timing.

When change detection sensor 601 detects change of contrast medium C flowing in chemical liquid injection tube 120 to physiological saline W, physiological saline W may be continuously fed to a predetermined volume before stop with pressure. In this case, since a predetermined volume of physiological saline W is injected into the patient after contrast medium C, contrast medium C can be appropriately fed to the body part of the patient to be imaged by physiological saline W.

When contrast medium C flowing in chemical liquid injection tube 120 is changed to physiological saline W and then physiological saline W is continuously fed to the predetermined volume with pressure to transfer contrast medium C to the body part to be imaged as described above, the continuously fed volume of physiological saline W depends on the body part to be imaged.

Thus, injection control unit 136 of chemical liquid injector 600 may be provided with a body part input means comprising keyboards or a touch panel for receiving entry of data of a body part to be imaged by the PET apparatus, a volume storing means comprising HDD (Hard Disc Drive) or flash memory for storing data of the volume of a liquid for each body part to be imaged, and a volume reading means comprising a logical circuit or a microcomputer for reading data of the volume in response to entry of data of the body part to be imaged. In this case, when the operator makes entry of data of the body part of the patient to be imaged, the volume of physiological saline W continuously fed with pressure is controlled on the basis of the body part to be imaged, so that contrast medium C can be appropriately injected into various body parts to be imaged.

When radioactive contrast medium C is injected into the patient and diagnostic images are taken by the PET apparatus or the like as described above, the imaging operation is started after a predetermined time period in which injected contrast medium C is absorbed in cells. Thus, the chemical liquid injector may be provided with a completion detection means for detecting the completion of injection of contrast medium C, a time detection means for detecting the lapse of the predetermined time period when the injection completion is detected, a data transmission means for transmitting data of the detected lapse of the time to the PET apparatus, and the like, by using a microcomputer, an I/F unit, and the like (not shown).

In this case, in response to the lapse of the predetermined time period after the injection of contrast medium C is completed, the chemical liquid injector transmits the data of the lapse of the time to the PET apparatus. The PET apparatus receives the data of the lapse of the time and starts imaging. In this manner, the imaging operation can be automatically started with contrast medium C appropriately absorbed in cells. It is possible that the chemical liquid injector immediately transmits data of the completion of injection of contrast medium C to the PET apparatus which then starts imaging operation after the detection of the lapse of a predetermined time period.

In the above embodiment, liquid pressure-feeding mechanism 131 feeds physiological saline W to chemical liquid container 200 from liquid container 300 through liquid supply tube 110 with pressure to feed contrast medium C to the patient from liquid container 200 through chemical liquid injection tube 120. If physiological saline W or contrast medium C may flow backward only the pressure applied by liquid pressure-feeding mechanism 131, as in chemical liquid injector 400 illustrated in Fig. 2, it is possible to insert one-way valve 430 into liquid supply tube 110 for regulating the flow direction of physiological saline W in one direction or to insert one-way valve (not shown) into chemical liquid injection tube 120 for regulating the flow direction of contrast medium C in one direction.

In the above embodiment, liquid pressure-feeding mechanism 131 has the peristaltic finger mechanism and the rotary pump mechanism to sequentially press liquid supply tube 110 connected to liquid container 300 to feed physiological saline W with pressure. Alternatively, it is possible to employ a liquid pressure-feeding mechanism (not shown) which is pushed a piston member with pressure into a cylinder member of a liquid syringe filled with physiological saline W to press physiological saline W into the liquid supply tube connected to that liquid syringe.

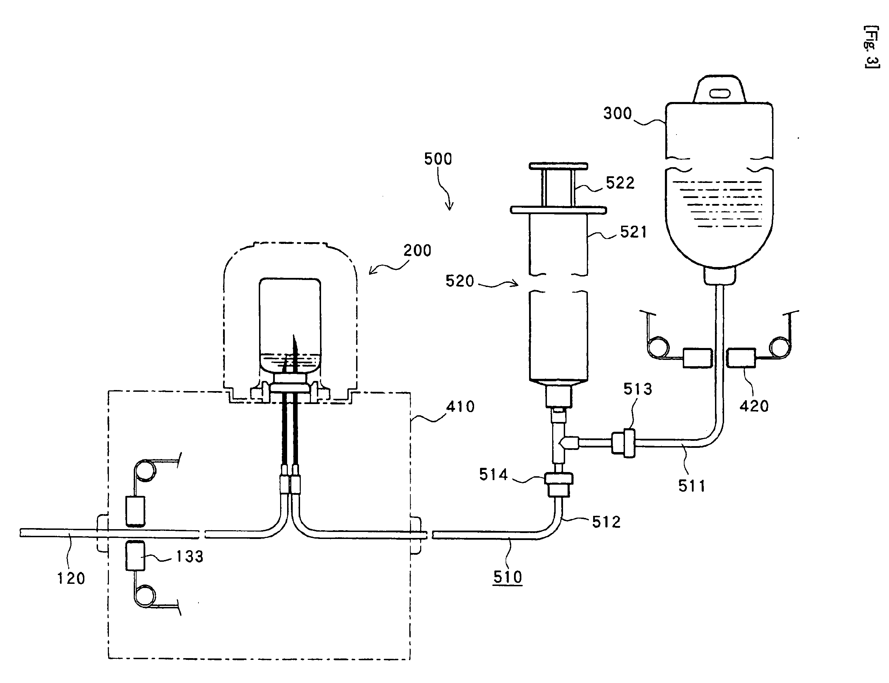

More specifically, in chemical liquid injector 500 shown in Fig. 3, liquid container 300 and liquid syringe 520 serving as a liquid pressure-feeding mechanism are connected to two trailing ends of bifurcated liquid supply tube 510. First/second one-way valves 513 and 514 are inserted into portion 511 of liquid supply tube 510 from liquid container 300 to liquid supply tube 510 and potion 512 of liquid supply tube 510 from liquid syringe 520 to chemical liquid container 200.

In chemical liquid injector 500, the flow of physiological saline W in liquid supply tube 510 is regulated in the direction from liquid container 300 to liquid syringe 520 by first one-way valve 513, and is regulated in the direction from liquid syringe 520 to chemical liquid container 200 by second one-way valve 514.

Liquid syringe 520 includes cylinder member 521 and piston member 522 slidably inserted into cylinder member 521, and piston member 522 is slid to cause cylinder member 521 to suck or discharge physiological saline W. Thus, in chemical liquid injector 500 illustrated in Fig. 3, each time liquid syringe 520 sucks and discharges, physiological saline W is fed to chemical liquid container 200 from liquid container 300 via liquid syringe 520 with pressure.

## Claims

1. A chemical liquid injection system comprising a chemical liquid container containing a chemical liquid and a chemical liquid injector injecting the chemical liquid into a patient,
wherein the chemical liquid injector includes:
a chemical liquid injection tube having a leading end connected to the patient and a trailing end placed within the chemical liquid container;
a liquid supply tube having a leading end placed within the chemical liquid container;
a liquid container containing a predetermined liquid and connected to a trailing end of the liquid supply tube; and
a liquid pressure-feeding mechanism feeding the liquid in the liquid container into the chemical liquid container from the liquid supply tube with pressure.

2. The chemical liquid injection system according to claim 1, wherein the trailing end of the chemical liquid injection tube is placed at a lower position within the chemical liquid container, and
the leading end of the liquid supply tube is placed at an upper position within the chemical liquid container.

3. The chemical liquid injection system according to claim 2, wherein the chemical liquid container includes a container body having an opening in a portion thereof and an elastic member sealing the opening of the container body,
the chemical liquid injector includes a chemical liquid holding mechanism holding the chemical liquid container such that the elastic member is located at the bottom,
the chemical liquid injection tube has a short tubular needle member formed at the trailing end, the short needle member being stuck through the elastic member, and
the liquid supply tube has a long tubular needle member formed at the leading end, the long needle member being stuck through the elastic member.

4. The chemical liquid injection system according to claim 2, wherein the chemical liquid container includes a container body having an opening in a portion thereof and an elastic member sealing the opening of the container body,
the chemical liquid injector includes a chemical liquid holding mechanism holding the chemical liquid container such that the elastic member is located at the top,
the chemical liquid injection tube has a long tubular needle member formed at the trailing end, the long needle member being stuck through the elastic member, and
the liquid supply tube has a short tubular needle member formed at the leading end, the short needle member being stuck through the elastic member.

5. The chemical liquid injection system according to claim 3 or 4, wherein the short needle member and the long needle member are formed integrally.

6. The chemical liquid injection system according to claim 2, wherein the chemical liquid container includes a container body having an opening in an portion thereof and a sealing member openably and closeably sealing the opening of the container body,
the chemical liquid injection tube has a short tubular member formed at the trailing end,
the liquid supply tube has a long tubular member formed at the leading end, and
the chemical liquid injector includes a tube holding member having the short tubular member and the long tubular member formed integrally and removably sealing the opening of the chemical liquid container, and a chemical liquid holding mechanism holding the chemical liquid container such that the opening is located at the bottom.

7. The chemical liquid injection system according to claim 2, wherein the chemical liquid container includes a container body having an opening in a portion thereof and a sealing member openably and closeably sealing the opening of the container body,
the chemical liquid injection tube has a long tubular member formed at the trailing end,
the liquid supply tube has a short tubular member formed at the leading end, and
the chemical liquid injector includes a tube holding member having the short tubular member and the long tubular member formed integrally and removably sealing the opening of the chemical liquid container, and a chemical liquid holding mechanism holding the chemical liquid container such that the opening is located at the top.

8. The chemical liquid injection system according to any one of claims 3 to 7, wherein the chemical liquid container includes a container body containing a radioactive contrast medium as the chemical liquid, and a container cover shielding radiation of the contrast medium, and
the chemical liquid injector includes a shield cover sealing at least part of the chemical liquid injection tube connected to the trailing end to shield the radiation, the shield cover being formed integrally with the chemical liquid holding mechanism.

9. The chemical liquid injection system according to any one of claims 2 to 8, wherein the chemical liquid container contains the liquid having a specific gravity smaller than that of the chemical liquid.

10. The chemical liquid injection system according to claim 1, wherein the trailing end of the chemical liquid injection tube is placed at a lower position within the chemical liquid container, and
the liquid container contains a predetermined liquid having a specific gravity smaller than that of the chemical liquid.

11. The chemical liquid injection system according to claim 9 or 10, wherein the chemical liquid container contains a contrast medium as the chemical liquid, and
the liquid container contains physiological saline as the liquid.

12. The chemical liquid injection system according to any one of claims 2 to 11, wherein the chemical liquid injector further comprises a bubble detection sensor detecting a bubble mixed in the chemical liquid flowing in the chemical liquid injection tube, and injection control means for forcedly stopping the liquid pressure-feeding mechanism when the bubble is detected.

13. The chemical liquid injection system according to any one of claims 2 to 12, wherein the chemical liquid injector further comprises a bubble detection sensor detecting a bubble mixed in the chemical liquid flowing in the chemical liquid injection tube, and an injection block mechanism blocking the flow of the chemical liquid in the chemical liquid injection tube when the bubble is detected.

14. The chemical liquid injection system according to any one of claims 2 to 13, wherein the chemical liquid injector further comprises a bubble removal mechanism removing a bubble from the chemical liquid flowing in the chemical liquid injection tube.

15. The chemical liquid injection system according to any one of claims 2 to 14, wherein the chemical liquid injector further comprises an exhaustion detection sensor detecting exhaustion of the liquid fed to the chemical liquid container from the liquid container through the liquid supply tube with pressure, and injection control means for forcedly stopping the liquid pressure-feeding mechanism when the exhaustion is detected.

16. The chemical liquid injection system according to any one of claims 2 to 15, wherein the chemical liquid injector further comprises an exhaustion detection sensor detecting exhaustion of the liquid fed to the chemical liquid container from the liquid container through the liquid supply tube with pressure, and a supply block mechanism blocking the flow of the liquid in the liquid supply tube when the exhaustion is detected.

17. The chemical liquid injection system according to any one of claims 2 to 16, further comprising a change detection sensor detecting a change of the chemical liquid to the liquid, the chemical liquid being fed to the patient from the chemical liquid container through the chemical liquid injection tube with pressure, and injection control means for controlling operation of the liquid pressure-feeding mechanism based on the detection of the change.

18. The chemical liquid injection system according to any one of claims 2 to 17, further comprising a change detection sensor detecting a change of the chemical liquid to the liquid, the chemical liquid being fed to the patient from the chemical liquid container through the chemical liquid injection tube with pressure, and injection control means for controlling operation of the liquid pressure-feeding mechanism based on the detection of the switch.

19. The chemical liquid injection system according to claim 17 or 18, wherein the chemical liquid container contains a radioactive contrast medium as the chemical liquid, and
the change detection sensor detects the change of the chemical liquid to the liquid based on the presence or absence of radiation.

20. The chemical liquid injection system according to any one of claims 17 to 19, wherein, when the change is detected, the injection control means forcedly stops the liquid pressure-feeding mechanism after a predetermined volume of the liquid is fed with pressure.

21. The chemical liquid injection system according to claim 20, further comprising a diagnostic imaging apparatus taking a diagnostic image of a body part of the patient where the chemical liquid is injected,
wherein the chemical liquid injector further comprises body part input means for receiving entry of data of a body part to be imaged by the diagnostic imaging apparatus, volume storing means for storing data of a volume of the liquid for each body part to be imaged, and volume reading means for reading data of the volume in response to entry of data of the body part to be imaged, and
when the change is detected, the injection control means forcedly stops the liquid pressure-feeding mechanism after the volume of the liquid is fed with pressure, the data of the volume being read.

22. The chemical liquid injection system according to any one of claims 2 to 21, further comprising a diagnostic imaging apparatus taking a diagnostic image of a body part of the patient where the chemical liquid is injected,
wherein the chemical liquid injector further comprises completion detection means for detecting completion of the injection of the chemical liquid, and data transmission means for transmitting data of the detected injection completion to the diagnostic imaging apparatus, and
the diagnostic imaging apparatus comprises imaging means for taking the diagnostic image, data receiving means for receiving the data of the injection completion, time detecting means for detecting the lapse of a predetermined time period when the data of the injection completion is received, and imaging control means for causing the imaging means to start imaging after the lapse of a predetermined time period.

23. The chemical liquid injection system according to any one of claims 2 to 21, further comprising a diagnostic imaging apparatus taking a diagnostic image of a body part of the patient where the chemical liquid is injected,
wherein the chemical liquid injector further comprises completion detection means for detecting completion of the injection of the chemical liquid, time detecting means for detecting the lapse of a predetermined time period when the injection completion is detected, and data transmitting means for transmitting data of the detected lapse of the time to the diagnostic imaging apparatus, and
the diagnostic imaging apparatus comprises imaging means for taking the diagnostic image, data receiving means for receiving the data of the lapse of the time, and imaging control means for causing the imaging means to start imaging when the data of the lapse of the time is received.

24. The chemical liquid injection system according to any one of claims 2 to 23, a one-way valve regulating a flow direction of the liquid in one direction is inserted into the liquid supply tube.

25. The chemical liquid injection system according to any one of claims 2 to 24, a one-way valve regulating a flow direction of the chemical liquid in one direction is inserted into the chemical liquid injection tube.

26. The chemical liquid injection system according to any one of claims 2 to 25, wherein the liquid container is replaceable.

27. A chemical liquid injector in the chemical liquid injection system according to claim 1, comprising:
a chemical liquid injection tube having a leading end connected to the patient and a trailing end placed within the chemical liquid container;
a liquid supply tube having a leading end placed within the chemical liquid container;
a liquid container containing a predetermined liquid and connected to a trailing end of the liquid supply tube; and
a liquid pressure-feeding mechanism feeding the liquid in the liquid container into the chemical liquid container from the liquid supply tube with pressure.

28. The chemical liquid injector according to claim 27, wherein the trailing end of the chemical liquid injection tube is placed at a lower position within the chemical liquid container, and
the leading end of the liquid supply tube is placed at an upper position within the chemical liquid container.

29. The chemical liquid injector according to claim 27, wherein the trailing end of the chemical liquid injection tube is placed at a lower position within the chemical liquid container, and
the liquid container contains a predetermined liquid having a specific gravity smaller than that of the chemical liquid.

30. The chemical liquid injector according to claim 28 or 29, further comprising:
a body part input means for receiving entry of data of a body part to be imaged by a diagnostic imaging apparatus taking a diagnostic image of a body part of the patient where the chemical liquid is injected;
volume storing means for storing data of a volume of the liquid for each body part to be imaged;
volume reading means for reading data corresponding to the volume in response to entry of data of the body part to be imaged; and
injection control means for forcedly stopping the liquid pressure-feeding mechanism after the volume of the liquid is fed with pressure when the change is detected, the data of the volume being read.

31. The chemical liquid injector according to claim 30, further comprising:
completion detection means for detecting completion of the injection of the chemical liquid; and
data transmission means for transmitting data of the detected injection completion to the diagnostic imaging apparatus.

32. The chemical liquid injector according to claim 30, further comprising:
completion detection means for detecting completion of the injection of the chemical liquid;
time detecting means for detecting the lapse of a predetermined time period when the injection completion is detected; and
data transmitting means for transmitting data of the detected lapse of the time to the diagnostic imaging apparatus.
